# EUROPEAN PATENT SPECIFICATION

(11) **EP 1 083 901 B1**
(45) Date of publication and mention of the grant of the patent: **16.04.2003**
(21) Application number: 99925793.4
(22) Date of filing: 24.05.1999
(51) Int. Cl.: A61K 31/535, A61K 9/20

(54) **EFAVIRENZ COMPRESSED TABLET FORMULATION**
EFAVIRENZ ENTHALTENDE GEPRESSTE TABLETTENFORMULIERUG
FORMULATION DE COMPRIME D'EFAVIRENZ

(30) Priority: 27.05.1998 US 86921 P; 21.07.1998 GB 9815800
(43) Date of publication of application: 21.03.2001
(73) Proprietor: MERCK & CO., INC., Rahway, New Jersey 07065 (US)
(72) Inventor: BATRA, Udit, Rahway, NJ 07065 (US); HIGGINS, Raymond, J., Rahway, NJ 07065 (US); THOMPSON, Karen, C., Rahway, NJ 07065 (US); KATDARE, Ashok, V., Rahway, NJ 07065 (US)
(74) Representative: Horgan, James Michael Frederic
(86) International application number: US9911464
(87) International publication number: WO99061026

(56) References cited:
- WO-A-95/20389
- WO-A-96/37457
- WO-A-98/52570

## Description

### BACKGROUND OF THE INVENTION

This invention relates to a compressed tablet formulation for efavirenz, which is 50 percent by weight drug loaded and can optionally be film coated. Efavirenz is a non-nucleoside reverse trancriptase inhibitor being studied clinically for use in the treatment of HIV infections and AIDS. A process for the manufacture of the compressed tablet is also disclosed.

The synthesis of efavirenz and structurally similar reverse transcriptase inhibitors are disclosed in US Patents 5,519,021, 5,663,169, 5,665,720 and the corresponding PCT International Patent Application WO 95/20389, which published on August 3, 1995. Additionally, the asymmetric synthesis of an enantiomeric benzoxazinone by a highly enantioselective acetylide addition and cyclization sequence has been described by Thompson, *et al*., Tetrahedron Letters *1995,* 36, 8937-8940, as well as the PCT publication, WO 96/37457, which published on November 28, 1996.

Additionally, several applications have been filed which disclose various aspects of the synthesis of(-)-6-chloro-4-cyclopropylethynyl-4-trifluoromethyl-1,4-dihydro-2H-3,1-benzoxazin-2-one including: 1) a process for making the chiral alcohol, U.S.S.N. 60/035,462, filed 14 January 1997; 2) the chiral additive, U.S.S.N. 60/034,926, filed 10 January 1997; 3) the cyclization reaction, U.S.S.N. 60/037,059, filed 12 February 1997; and the anti-solvent crystallization procedure, U.S.S.N. 60/037,385 filed 5 February 1997 and U.S.S.N. 60/042,807 filed 8 April 1997.

The compressed tablet is an improved formulation which allows one to utilize a tablet over a capsule. The compressed tablet has been demonstrated to have comparable bioavailability data to that seen with the capsule. The key feature of the formulation is the use of a superdisintegrant and disintegrant intragranularly to achieved a bioequivalent formulation. The compressed tablet form was difficult to manage as efavirenz is fragile and the drug loses crystallinity upon compression. This was overcome by adding lactose extragranularly.

### SUMMARY OF THE INVENTION

The instant invention relates to a compressed tablet of efavirenz which is a 50 percent drug loaded formulation.

The instant invention also relates to the process for manufacture of the compressed tablet using a wet granulation method.

### DETAILED DESCRIPTION OF THE INVENTION

The instant invention relates to a compressed tablet of efavirenz formulation which is 50 percent by weight drug loaded and can optionally be film coated.

The compressed tablet comprises: efavirenz, filler/disintegrant, superdisintegrant, binder, surfactant, filler/compression aid, lubricant, and solvent, wherein of efavirenz is about 50% by weight of the total composition of the compressed tablet.

The efavirenz concentration can be varied from about 1 to about 75 % by changing the concentration of remaining excipients. Furthermore, changing the tooling can give a wide ranges of doses, e.g. a 20 mg dose in a 40 mg tablet, a 300 mg dose in a 600 mg tablet,or a 600 mg dose in a 1200 mg compressed tablet, with the same composition. Removing the lactose from the formulation gives about 70 % drug in the formulation giving a 600 mg dose in a 860 mg compressed tablet. These variations are very straightforward to effect. This formulation will allow one to formulate efavirenz as a single 600 mg dose as an 860 mg compressed tablet, where as a capsule formulation requires the administration of at least two capsules to dose with 600 mg of efavirenz.

The invention contemplates the use of any pharmaceutically acceptable fillers/compression aids, disintegrants, super-disintegrants, lubricants, binders, surfactants, film coatings, and solvents. Examples of these components are set forth below and are described in more detail in the Handbook of Pharmaceutical Excipients, Second Edition, Ed. A. Wade and P.J. Weller, 1994, The Pharmaceutical Press, London, England.

Fillers and compression aid concentrations can be varied between about 5% to about 80% to complement the drug amount. Examples of fillers/compression aids include: lactose, calcium carbonate, calcium sulfate, compressible sugars, dextrates, dextrin, dextrose, calcium phosphate, kaolin, magnesium carbonate, magnesium oxide, maltodextrin mannitol, powdered cellulose, pregelatinized starch, and sucrose.

Examples of disintegrants include: alginic acid, carboxymethylcellulose calcium, carboxymethylcellulose sodium, colloidal silicon dioxide, croscarmellose sodium, crospovidone, guar gum, magnesium aluminum silicate, methylcellulose, microcrystalline cellulose, polyacrilin potassium, powdered cellulose, pregelatinized starch, sodium alginate and starch.

Examples of fillers (also referred to as a diluent) include: calcium carbonate, calcium sulfate, compressible sugars, confectioner's sugar, dextrates, dextrin, dextrose, dibasic calcium phosphate dihydrate, glyceryl palmitostearate, hydrogenated vegetable oil (type I), kaolin, lactose, magnesium carbonate, magnesium oxide, maltodextrin, mannitol, polymethacrylates, potassium chloride, powdered cellulose, pregelatinized starch, sodium chloride, sorbitol, starch, sucrose, sugar spheres, talc and tribasic calcium phosphate.

Superdisintegrant concentration can be varied between about 1% to about 20% to complement the drug amount and obtain reasonable dissolution. Examples of super-disintegrants include the disintegrants listed above, carboxymethylcellulose sodium, croscarmellose sodium, povidone, guar gum, polacrilin potassium, and pregelatinized starch.

Binder concentration can be varied between 1 and 10 % to complement the drug amount. Examples of binders include: acacia, alginic acid, carbomer, carboxymethylcellulose sodium, dextrin, ethylcellulose, gelatin, guar gum, hydrogenated vegetable oil (type I), hydroxyethyl cellulose, hydroxypropyl cellulose, hydroxypropyl methylcellulose, liquid glucose, magnesium aluminaum silicate, maltodextrin, methylcellulose, polymethacrylates, povidone, pregelatinized starch, sodium alginate, starch, and zein.

Examples of surfactants comprises anionic and cationic surfactants, such as sodium lauryl sulfate, docusate sodium (dioctyl sulfosuccinate sodium salt), benzalkonium chloride, benzethonium chloride, and cetrimide (alkyltrimethylammonium bromide, predominantly C₁₄ alkyl).

Examples of lubricants include: calcium stearate, glyceryl monostearate, glyceryl palmitostearate, hydrogenated castor oil, hydrogenated vegetable oil, light mineral oil, magnesium stearate, mineral oil, polyethylene glycol, sodium benzoate, sodium lauryl sulfate, sodium stearyl fumarate, stearic acid, talc and zinc stearate.

Examples of solvent comprises: water, ethanol or mixtures thereof.

The compressed tablet can also be film coated. Film coat concentration can be varied up to about 10 % to complement the drug amount, and preferably about 3.1% to about 3.3%. Film coating suspensions include combinations of one, two or three of the following components: carboxymethylcellulose sodium, carnauba wax, cellulose acetate phthalate, cetyl alcohol, confectioner's sugar, ethyl cellulose, gelatin, hydroxyethyl cellulose, hydroxypropyl cellulose, hydroxypropyl methylcellulose, liquid glucose, maltodextrin, methyl cellulose, microcrystalline wax, Opadry and Opadry II, polymethacrylates, polyvinyl alcohol, shellac, sucrose, talc, titanium dioxide, and zein.

The preferred filler/disintegrant is microcrystalline cellulose. The preferred superdisintegrant is croscarmellose sodium. The preferred binder is hydroxypropyl cellulose. A preferred surfactant is sodium lauryl sulfate. The preferred diluent/compression aid is lactose hydrous spray dried. The preferred lubricant is magnesium stearate. The preferred solvent for formulating this compressed tablet is water. The preferred film coating comprises: hydroxypropylcellulose, hydroxypropyl methylcellulose, and titanium dioxide.

The 300 mg film coated efavirenz tablet contains:

| **Ingredient** | **Amt per tablet** | **Percent w/w** |
|---|---|---|
| **Core Tablet:** | | |
| efavirenz | 300 mg | 50 |
| microcrystalline cellulose NF | 120 mg | 20 |
| hydroxypropyl cellulose LF NF | 19.2 mg | 3.2 |
| croscarmellose sodium | 30 mg | 5 |
| sodium lauryl sulfate | 6 mg | 1 |
| lactose hydrous spray dried (EG) | 118.8 mg | 19.8 |
| magnesium stearate (EG) | 6 mg | 1 |
| **Film Coating Material per Tablet:** | 3.1% by wt | |
| hydroxypropyl cellulose LF NF | 8.05 mg | 1.4 |
| hydroxypropyl methylcellulose USP 6CPS | 8.05 mg | 1.4 |
| titanium dioxide USP | 3.1 mg | 0.3 |
| Tablet Weight: | 619.2 mg | |

A process for the preparation of a 50 % drug loaded compressed tablet comprising the following steps:
(a) blending efavirenz with a filler/disintegrant, super-disintegrant, binder and surfactant;
(b) adding at least 1.1% by weight of water per weight of efavirenz to wet granulate the blended mixture to agglomerate the mixture;
(c) drying the granulated mixture to a moisture content of about 0% to about 10%;
(d) milling the dried mixture to granulate to a uniform size;
(e) blending the milled mixture with a filler/compression aid;
(f) lubricating the blended mixture with a lubricant; and
(g) compressing the lubricated mixture to a compressed tablet of the desired shape.

The process as recited above which comprises the additional step of film coating the compressed tablet with a film coating suspension to produce the desired film coated compressed tablet.

The process as recited above wherein the granulated mixture is dried to a moisture contant of about 2% to about 5%.

A process for the preparation of a 50 % drug loaded compressed tablet comprising the following steps:
(a) blending efavirenz with microcrystalline cellulose, sodium lauryl sulfate, hydroxypropyl cellulose and croscarmellose sodium;
(b) adding at least 1.1 weight % water per weight of efavirenz to wet granulate the blended mixture for about 3 minutes to about 8 minutes to agglomerate the mixture;
(c) drying the granulated mixture to a moisture content of about 2% to about 5%;
(d) milling the dried mixture to a granulate of about 250µ to about 75µ;
(e) blending the milled mixture with lactose;
(f) lubricating the blended mixture with magnesium stearate;
(g) compressing the lubricated mixture to a compressed tablet of the desired shape; and
(h) film coating the compressed tablet with a film coating suspension to about 3.1% to about 3.3% of weight of compressed tablet.

The process as recited above wherein the blended mixture is wet granulated for about 6 minutes.

The process as recited above wherein the film coating suspension comprising hydroxypropylcellulose, hydroxypropyl methylcellulose, and titanium dioxide.

Wet granulation can be conducted using granulator mixers, such as a Fielder 10 L high shear granulator mixer, a drum or pan granulator,and a fluid bed granulator. Granulation can also be achieved by conducting dry granulation (without water) using a roller compaction process.

The drying step can be conducted using a Glatt WST-15 fluid bed drier or a tray drier.

The milling step can be conducted using mills such as a Comil or a Fitz mill.

The lubricating and blending steps can be conducted in a V-blender or a ribbon blender.

The compression step to form the tablet can be done a variety of presses including a beta press, single station F-press, the 6-station Korsh, etc.

Film coating can be performed in a Glatt Column coater, a smaller Hi-coater (9"- 12 " pan), etc.

The formulation also is bioequivalent to a capsule with a smaller dose (200 mg), and more bioavailable than other tablet compositions. The advantages over the capsule include robust processing and sorting steps, smaller size with a larger dose, and market preference. The tablet composition also overcomes the expect loss of crystallinity of efavirenz by adding the lactose extra-granularly while maintaining the dissolution profile.

The increased drug loading often compromises the dissolution profile of the drug. This hurdle was overcome by including the super-disintegrant intragranularly, as well as the disintegrant intragranularly. The lactose was added extra-ganularly to maintain the crystallinity of efavirenz.

This formulation was determined to be bioequivalent to the capsule formulation being used in clinical trials. The wet granulation process has been used to optimize the formulation such that about 80% dissolution of the drug occurs within 10 minutes in a 1% Sodium Dodecyl sulfate (SDS) solution, while stirring at a 50 rpm paddle speed.

### Preparation of (-)-6-chloro-4-cyclopropylethynyl-4-trifluoromethyl-1,4-dihydro-2H-3,1-benzoxazin-2-one (currently referred to by its generic name efavirenz or code name DMP-266).

Scheme 1 outlines the key steps in the synthesis of (-)-6-chloro-4-cyclopropylethynyl-4-trifluoromethyl-1,4-dihydro-2H-3,1-benzoxazin-2-one (efavirenz). The chiral addition step allows for the enantioselective addition of the cyclopropylacetylide across the trifluoromethylketone of **1**. The p-methoxybenzyl (PMB)-protected amino alcohol, **2**, produced is then deprotected to give the amino alcohol, **3**. The amino alcohol is then cyclized using a chloroformate and base to give efavirenz.

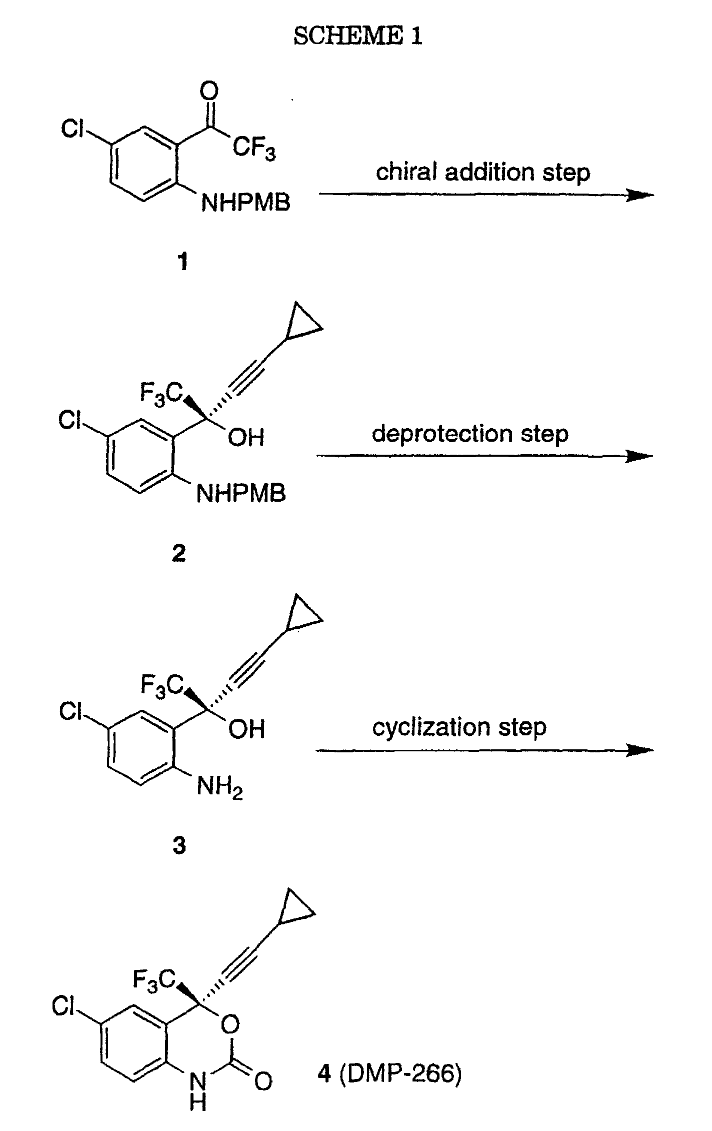

Scheme 2 outlines the preparation of efavirenz using an alternative process which is a chiral addition reaction. The new chiral addition reaction allows for the elimination of the protection-deprotection sequence as outlined in Scheme 1.

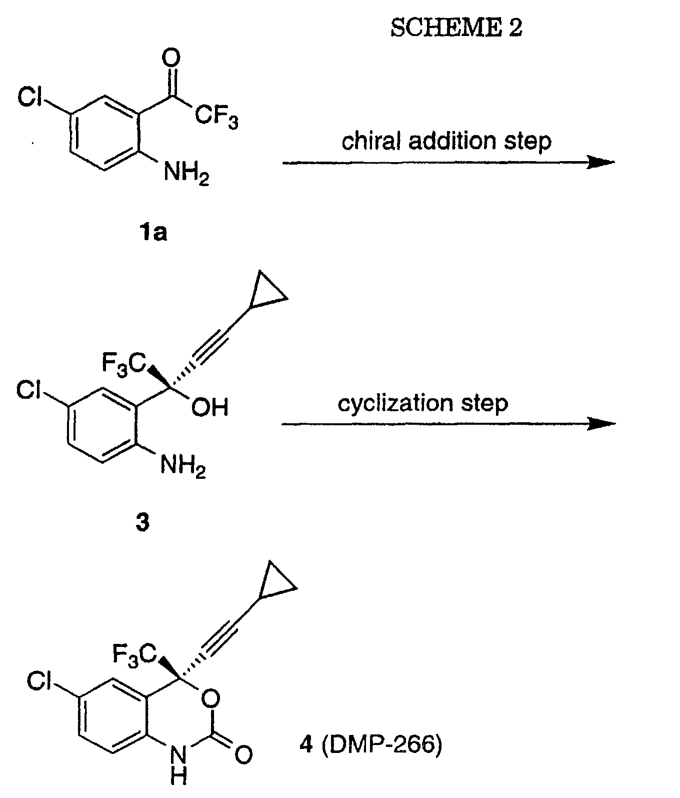

Scheme 3 describes the process for the synthesis of the chiral intermediate used in the preparation of efavirenz. This reaction has been demonstrated to work using about 1.2 equivalents of cyclopropylacetylene and chiral additive, much lesss than the prior methods. The numerous chiral additives have been run and give high yields with a commerically available chiral ligand, such as N-methyl ephedrine and N-pyrrolidinyl norephedrine.

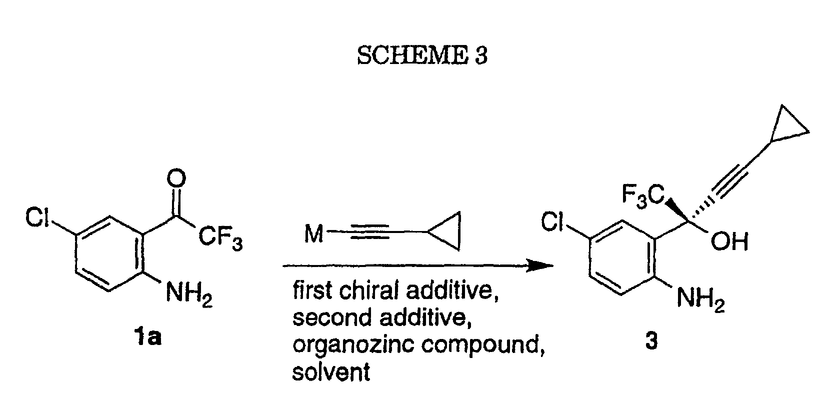

The cyclization of the amino alcohol, **3** to produce the 1,4-dihydro-2H-3,1-benzoxazin-2-one, **4** is outlined in Scheme 4 below. The reaction can be carried out as a one-step process, or alternatively a two step process with the potential isolation of the intermediate carbamate, **5** depending upon the chloroformate utilized. It has been demonstrated that the aryl chloroformates form less stable carbamates such that when they are treated with aqueous base they cyclize to the product, in a one-step process. The alkyl chloroformate, alternatively, provides an alkyl carbamate, a key intermediate capable of being isolated and purified prior to carrying out the cyclization step. Based upon the stability of the alkyl carbamates, a viable two step process for the preparation of efavirenz has been developed which comprises the formation of the alkyl carbamate intermediate, **5** followed by the cyclization of the carbamate to give the desired product, **4**. Additionally, it has been demonstrate that phosgene can also be used.

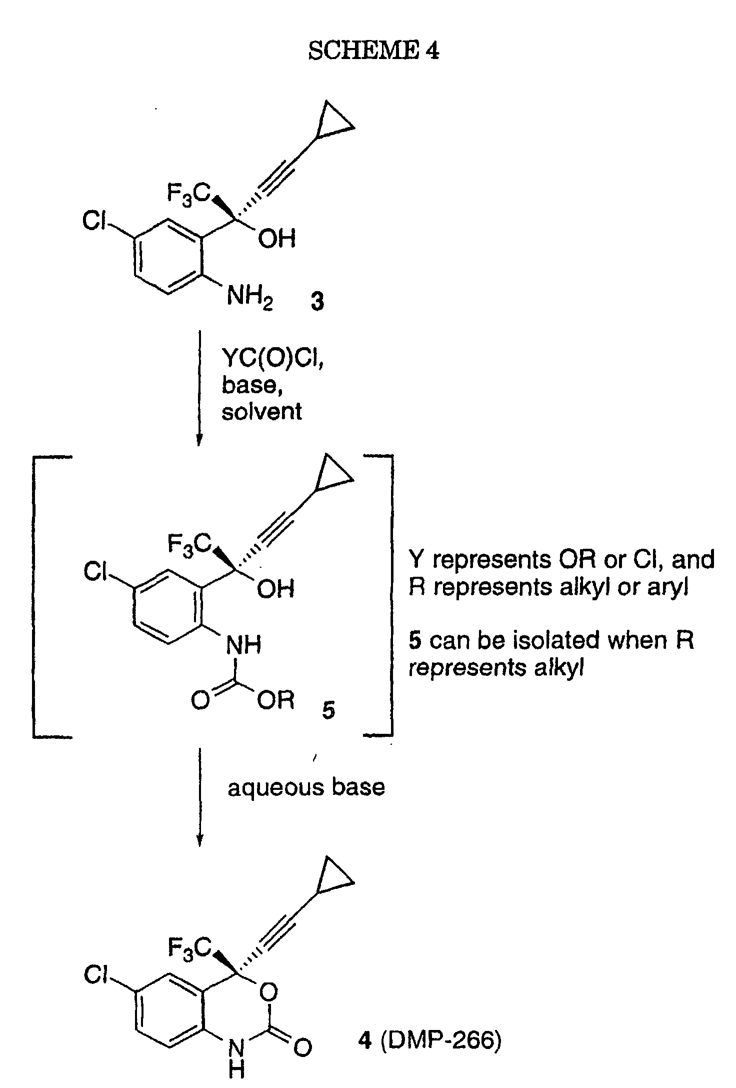

The compressed tablet is formulated following the sequence of steps outlined in Scheme 5.

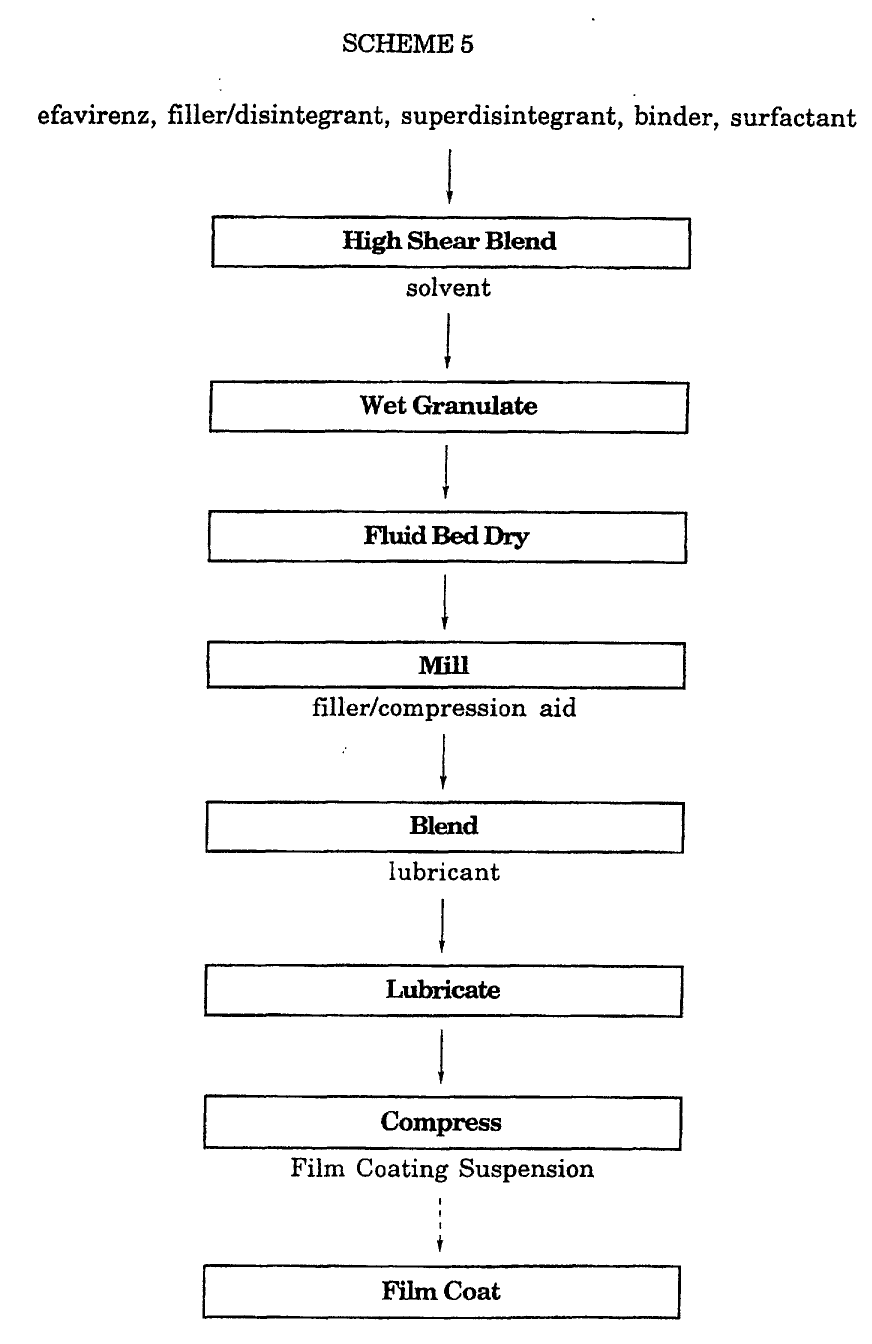

The following examples are meant to be illustrative of the present invention. These examples are presented to exemplify the invention and are not to be construed as limiting the scope of the invention.

### EXAMPLE 1

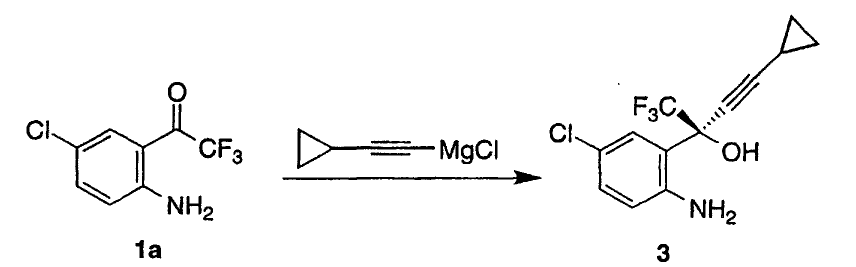

| Materials | Amount | Mol | MW |
|---|---|---|---|
| Ketone **1a** | 1.00 kg g | 4.47 | 223.58 |
| (1R, 2S)-N-pyrrolidinyl norephedrine | 1.35 kg | 6.58 | 205.30 |
| cyclopropyl acetylene | 361.9 g | 5.47 | 66.10 |
| *n*-BuMgCl (2.0 M in THF) , | 2.68 L | 5.37 | |
| 2,2,2-trifluoroethanol (99%) | 429.5 g | 4.29 | 100.04 |
| ZnEt₂ (0.892 M in hexane) | 6.02 L | 5.37 | |
| THF | 9.36 L | | |
| 30% K₂CO₃ | 550 mL | | |
| 30% citric acid | 2.0 L | | |
| Toluene (for crystallization, 2 mL/g of **4**) | 2.6 L | | |
| Heptane (for crystallization, 4 mL/g of **4**) | 5.2 L | | |

To a solution of trifluoroethanol and (1R, 2S)-N-pyrrolidinyl norephedrine in THF (9 L) under nitrogen is added a solution of diethylzinc in hexane at 0 °C slowly enough to keep the temperature below 30 °C. The mixture is stirred at room temperature for 0.5 ∼ 1 h. In another dry flask a solution of chloromagnesium cyclopropyl acetylide is prepared as follows: To neat cyclopropyl acetylene at 0 °C is added a solution of *n*-butylmagnesium chloride slowly enough to keep the internal temperature ≤ 30 °C. The solution is stirred at 0 °C for ∼ 40 min and transfered to the zinc reagent *via* cannula with 0.36 L of THF as a wash. The mixture is cooled to -10 °C and ketoaniline **1a** is added. The mixture is stirred at -2 to -8 °C for 35 h, warmed to room temperature, stirred for 3 h, and quenched with 30% potassium carbonate over 1.5 h. The mixture is stirred for 4 h and the solid is removed by filtration and washed with THF (2 cake volume). The wet solid still contains ∼18 wt% of pyrrolidinyl norephedrine and is saved for further study. The filtrate and wash are combined and treated with 30% citric acid. The two layers are separated. The organic layer is washed with water (1.5 L). The combined aqueous layers are extracted with 2.5 L of toluene and saved for norephedrine recovery. The toluene extract is combined with the organic solution and is concentrated to ∼ 2.5 L. Toluene is continuously feeded and distilled till THF is not detectable by GC. The final volume is controlled at 3.9 L. Heptane (5.2 L) is added over 1 h. The slurry is cooled to 0 °C, aged for 1 h, and filtered. The solid is washed with heptane (2 cake volume) and dried to give 1.234 Kg (95.2% yield) of amino alcohol **3** as a white crystalline. The material is 99.8 A% pure and 99.3% ee.

### EXAMPLE 2

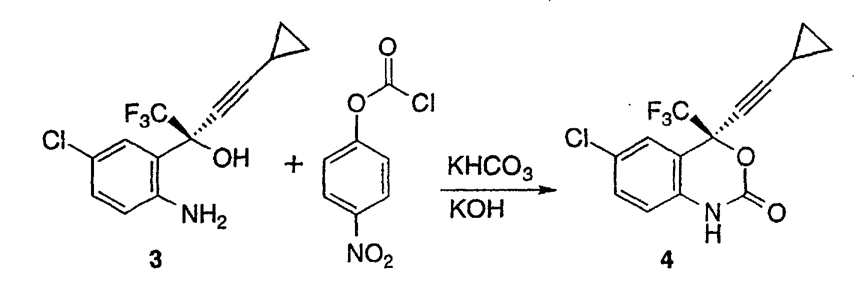

| | FW | g | mL | mmol | equiv |
|---|---|---|---|---|---|
| amino alcohol **3** | 289 | 100 | | 346 | 1 |
| 4-nitrophenylchloroformate | 201.6 | 73.2 | | 363 | 1.05 |
| KHCO₃ | 100 | 45 | | 450 | 1.3 |
| 2N KOH | 56 | | 346 | 692 | 2.0 |
| H₂O | | | 654 | | |
| MTBE | | | 500 | | |

To a three necked round bottom flask, equipped with a mechanical stirrer, nitrogen line, and thermocouple, was charged the solid amino alcohol **3**, MTBE (500 mL), and aqueous KHCO₃ (45 g in 654 mL H₂O). Solid 4-nitrophenyl chloroformate was added, in 4 batches, at 25°C. During the addition the solution pH was monitored. The pH was maintained between 8.5 and 4 during the reaction and ended up at 8.0. The mixture was stirred at 20-25°C for two hours. Aqueous KOH (2N) was added over 20 minutes, until the pH of the aqueous layer reached 11.0.

The layers were separated and 500 mL brine was added to the MTBE layer. 0.1 N Acetic acid was added until the pH was 6-7. The layers were separated and the organic phase was washed with brine (500 mL). At this point the mixture was solvent switched to EtOH/IPA and crystallized as recited in Examples 5 and 6.

### EXAMPLE 3

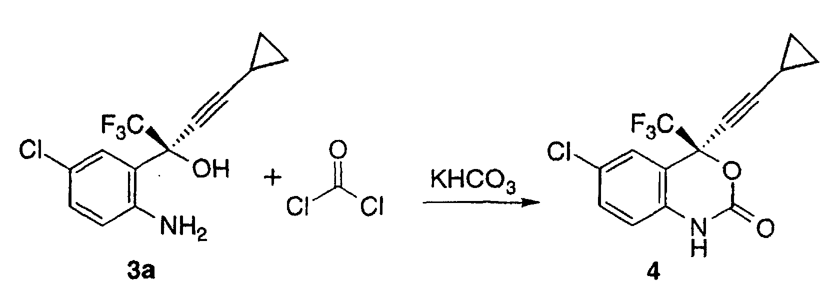

| | FW | g | mL | mmol | equiv |
|---|---|---|---|---|---|
| amino alcohol **3a** | 289 | 100 | | 346 | 1 |
| phosgene (20 wt% in toluene) | 99 | 41 | 216 | 415 | 1.2 |
| KHCO₃ | 100 | 86.5 | | 865 | 2.5 |
| H₂O | | | 500 | | |
| Toluene | | | 500 | | |

To a three necked round bottom flask, equipped with a mechanical stirrer, nitrogen line, and thermocouple, was charged the solid amino alcohol **3a**, toulene (500 mL), and aqueous KHCO₃ (86.5 g in 500 mL H₂O). Phosgene solution in toulene was added at 25°C, and the mixture was stirred at 20-25°C for two hours.

The layers were separated and the organic phase was washed with brine (500 mL). At this point the mixture was solvent switched to EtOH/IPA and crystallized as recited in Examples 5 and 6.

### EXAMPLE 4

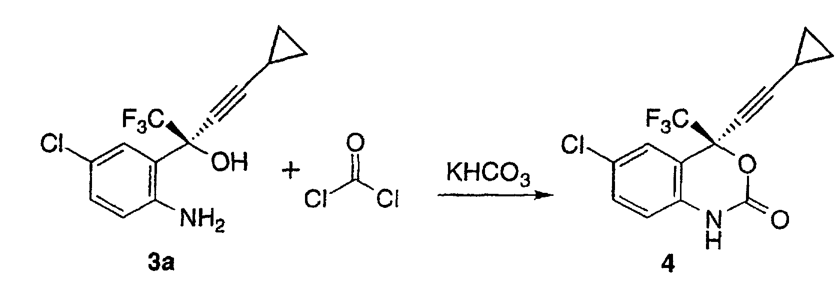

| | FW | g | mL | mmol | equiv |
|---|---|---|---|---|---|
| amino alcohol **3a** | 289 | 100 | | 346 | 1 |
| phosgene (gas) | 99 | | | | |
| KHCO₃ | 100 | 86.5 | | 865 | 2.5 |
| H₂O | | | 500 | | |
| MTBE | | | 500 | | |

To a three necked round bottom flask, equipped with a mechanical stirrer, nitrogen line, and thermocouple, was charged the solid amino alcohol **3a**, MTBE (500 mL), and aqueous KHCO₃ (86.5 g in 500 mL H₂O). Phosgene gas was slowly passed into the solution at 25°C, until the reaction was complete.

The layers were separated and the organic phase was washed with brine (500 mL). At this point the mixture was solvent switched to EtOH/IPA and crystallized as recited in Examples 5 and 6.

### EXAMPLE 5

Crystallization of efavirenz from 30% 2-Propanol in Water using a ratio of 15 ml solvent per gram efavirenz Using Controlled Anti-Solvent Addition on a 400 g Scale.

400 g. of efavirenz starting material is dissolved in 1.8 L of 2-propanol. The solution is filtered to remove extraneous matter. 1.95 L of deionized (DI) water is added to the solution over 30 to 60 minutes. 10 g. to 20 g. of efavirenz seed (Form II wetcake) is added to the solution. The seed bed is aged for 1 hour. The use of Intermig agitators is preferred to mix the slurry. If required (by the presence of extremely long crystals or a thick slurry), the slurry is wet-milled for 15 - 60 seconds. 2.25 L of DI water is added to the slurry over 4 to 6 hours. If required (by the presence of extremely long crystals or a thick slurry), the slurry is wet-milled for 15 - 60 seconds during the addition. The slurry is aged for 2 to 16 hours until the product concentration in the supernatant remains constant. The slurry is filtered to isolate a crystalline wet cake. The wet cake is washed with 1 to 2 bed volumes of 30 % 2-propanol in water and then twice with 1 bed volume of DI water each. The washed wet cake is dried under vacuum at 50°C.

### EXAMPLE 6

Crystallization of efavirenz from 30% 2-Propanol in Water using a ratio of 15 ml solvent per gram efavirenz Using a Semi-Continuous Process on a 400 g Scale.

400 g. of efavirenz starting material is dissolved in 1.8 L of 2-propanol. A heel slurry is produced by mixing 20 g. of Form II efavirenz in 0.3 L of 30 % (v/v) 2-propanol in water or retaining part of a slurry froma previous crystallization in the crystallizer. The dissolved batch and 4.2 L of DI water are simultaneously charged to the heel slurry at constant rates over 6 hours to maintain a constant solvent composition in the crystallizer. Use of Intermig agitators during the crystallization is preferred. During this addition the slurry is wet-milled when the crystal lengths become excessively long or the slurry becomes too thick. The slurry is aged for 2 to 16 hours until the product concentration in the supernatant remains constant. The slurry is filtered to isolate a crystalline wet cake. The wet cake is washed with 1 to 2 bed volumes of 30 % 2-propanol in water and then twice with 1 bed volume of DI water each. The washed wet cake is dried under vacuum at 50°C.

### EXAMPLE 7

### Preparation of Amino Alcohol 3 and ee Upgrading-- Through Process

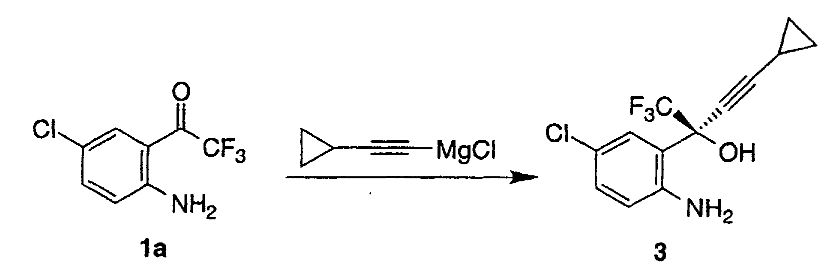

| Materials | Amount | Mol | MW |
|---|---|---|---|
| Ketone **1** | 1.00 kg | 4.47 | 223.58 |
| (1R, 2S)-N-Pyrrolidinyl norephedrine | 1.35 kg | 6.58 | 205.30 |
| Cyclopropyl acetylene | 361.9 g | 5.47 | 66.10 |
| *n*-BuMgCl (2.0 M in THF) | 2.68 L | 5.37 | |
| Trifluoroethanol (99%) | 429.5 g | 4.29 | 100.04 |
| ZnEt₂ (0.892 M in hexane) | 6.02 L | 5.37 | |
| THF | 9.36 L | | |
| 30% K₂CO₃ | 1.2 L | | |
| I M Citric acid | 3.5 L | | |
| Heptane | 12 L | | |
| Isopropyl acetate (IPAc) | 40 L | | |
| 12N HCl | 405 mL | 4.88 | |
| *tert*-Butyl methyl ether (MTBE) | 6 L | | |
| Toluene | 6.25 L | | |
| Na₂CO₃ | 1.2 kg | 11.25 | |

A solution of diethyl zinc in hexane was added to a solution of trifluoroethanol (429.5 g, 4.29'mol) and (1R, 2S)-N-pyrrolidinyl norephedrine (1.35 kg, 6.58 mol) in THF (9 L), under nitrogen, at 0 °C. The resulting mixture was stirred at room temperature for approx. 30 min. In another dry flask a solution of chloromagnesiumcyclopropylacetylide was prepared as follows. To a solution of *n*-butylmagnesium chloride in THF (2 M, 2.68 L, 5.37 mol) was added neat cyclopropylacetylene at 0 °C keeping the temperature ≤ 25 °C. The solution was stirred at 0 °C for 1 ∼ 2 h. The solution of chloromagnesiumcyclopropylacetylide was then warmed to room temperature and was transferred into the zinc reagent *via* cannula over 5 min followed by vessel rinse with 0.36 L of THF. The resulting mixture was aged at ∼ 30 °C for 0.5 h and was then cooled to 20 °C. The ketoaniline 1 (1.00 kg, 4.47 mol) was added in one portion as a solid, and the resulting mixture was stirred at 20-28 °C for 3 h.

The reaction was quenched with 30% aq. potassium carbonate (1.2 L) and aged for 1 h. The solid waste was filtered and the cake was washed with THF (3 cake volumes). The filtrate and wash were combined and solvent switched to IPAc.

The IPAc solution of product **3** and pyrrolidinyl norephedrine was washed with citric acid (3.5 L) and with water (1.5 L). The combined aqueous layers were extracted with IPAc (2 L) and saved for norephedrine recovery. To the combined organic layers was added 12N HCl (405 mL, 4.88 mol), to form a thin slurry of the amino alcohol-HCl salt. The mixture was aged for 30 min at 25 °C and was then dried azeotropically.

The slurry was aged at 25 °C for 30 min and filtered. The cake was washed with 2.5 L of IPAc and dried at 25 °C under vacuum/nitrogen for 24 h to give 1.76 kg of the wet HCl salt.

The salt was dissolved in a mixture of MTBE (6 L) and aq Na₂CO₃ (1.18 kg in 6.25 L water). The layers were separated and the organic layer was washed with 1.25 L of water. The organic layer was then solvent switched into toluene.

Heptane (5 L) was added over 1 h at 25 °C. The slurry was cooled to 0 °C, aged for 1 h, and filtered. The solid was washed with heptane (2 cake volumes) and was dried to give 1.166 kg (90% overall yield) of amino alcohol **3** as a white crystalline solid. Norephedrine recovery

The aqueous solution was basified to pH13 using 50% aq NaOH, and extracted with heptane (2 L). The heptane solution was washed with water (1 L) and concentrated to remove residual IPAc and water. The final volume was adjusted to about 3 L. The heptane solution was cooled to -20 °C, aged for 2 h, and filtered. The solid was washed with cold heptane (1 cake volume) and dried to give 1.269 kg solid (94% recovery).

### EXAMPLE 8

| 50 % Drug-Loaded Compressed tablet Of Efavirenz | |
|---|---|
| **Ingredient** | **Amt per batch** |
| **Core Tablet:** | |
| efavirenz | 950 g |
| microcrystalline cellulose NF | 380 g |
| hydroxypropyl cellulose LF NF | 60.8 g |
| croscarmellose sodium | 95 g |
| sodium lauryl sulfate | 19 g |
| lactose hydrous spray dried (EG)* | 19.8 % w/w |
| magnesium stearate (EG)* | 1% w/w |
| water | 1.045 L |
| **Film Coating Material per Tablet:** | 3.3 % by wt of tablet |
| hydroxypropyl cellulose LF NF | 8.54 mg (2.5 %) |
| hydroxypropyl methylcellulose USP 6CPS | 8.54 mg (2.5 %) |
| titanium dioxide USP | 3.42 mg (1 %) |
| water | (94 %) |

| | |
|---|---|
| * EG = extragranular | |

Efavirenz (950 g) was blended with microcrystalline cellulose (380 g), sodium lauryl sulfate (19 g), hydroxypropyl cellulose (60.8 g) and croscarmellose sodium (95 g) in a Fielder 10 L high shear granulator mixer for four minutes. At least about 1.1 weight % water per weight of efavirenz (1.045 L) was added to wet granulate the blended mixture over about 6 minutes to about 8 minutes to agglomerate the mixture using an appropriate spray nozzle. The granulated mixture is dried to a moisture content of about 2% to about 5% in a Glatt WST-15 fluid bed drier. The dried mixture was milled using a 40 G round screen in a Comil. The milled mixture was blended in a V-Blender with lactose for 4 minutes (calculated amount is the amount needed to make the final composition contain 19.8 % lactose by weight). The blended mixture was lubricated with magnesium stearate (calculated amount is the amount needed to make the final composition contain 1 % magnesium stearate by weight) in the V-Blender for 3 minutes. The lubricated mixture was compressed using a beta press to give a compressed tablet of the desired shape. The compressed tablets were film coated with an aqueous coating suspension that contains 2.5 % hydroxypropyl cellulose (HPC); 2.5 % hydroxymethylcellulose (HPMC); and 1 % titanium dioxide (TiO₂) and 94 % water by weight percent in a 19" O'Hara pan coater to a coat weight of about 3.3% per tablet. Note that the coat is the dried form of the suspension.

## Claims

1. A compressed tablet comprising: efavirenz, filler/disintegrant, superdisintegrant, binder, surfactant, diluent/compression aid, lubricant, and solvent, wherein efavirenz is crystalline and is from 1 to 75% by weight of the total composition of the compressed tablet.

2. The compressed tablet, as recited in Claim 1, wherein the filler comprises: lactose, calcium carbonate, calcium sulfate, compressible sugars, dextrates, dextrin, dextrose, calcium phosphate, kaolin, magnesium carbonate, magnesium oxide, maltodextrin mannitol, powdered cellulose, pregelatinized starch, or sucrose.

3. The compressed tablet, as recited in Claim 1 or 2, wherein the disintegrant and superdisintegrant comprise: alginic acid, carboxymethylcellulose calcium, carboxymethylcellulose sodium, colloidal silicon dioxide, croscarmellose sodium, crospovidone, guar gum, magnesium aluminium silicate, methylcellulose, microcrystalline cellulose, polyacrilin potassium, powdered cellulose, pregelatinized starch, sodium alginate or starch.

4. The compressed tablet, as recited in Claim 1, 2 or 3, wherein the binder comprises: acacia, alginic acid, carbomer, dextrin, ethylcellulose, gelatin, guar gum, hydroxyethyl cellulose, hydroxypropyl cellulose, hydroxypropyl methylcellulose, liquid glucose, magnesium aluminium silicate, maltodextrin, methylcellulose, polymethacrylates, povidone, pregelatinized starch, sodium alginate, starch, or zein.

5. The compressed tablet, as recited in Claim 1, 2, 3 or 4, wherein the surfactant comprises: sodium lauryl sulfate, docusate sodium, benzalkonium chloride, benzethonium chloride, or cetrimide.

6. The compressed tablet, as recited in any one of Claims 1 to 5, wherein the diluent/compression aid comprises: calcium carbonate, calcium sulfate, compressible sugars, confectioner's sugar, dextrates, dextrin, dextrose, dibasic calcium phosphate dihydrate, glyceryl palmitostearate, hydrogenated vegetable oil (type I), kaolin, lactose, such as lactose hydrous spray dried, magnesium carbonate, magnesium oxide, maltodextrin, mannitol, polymethacrylates, potassium chloride, powdered cellulose, pregelatinized starch, sodium chloride, sorbitol, starch, sucrose, sugar spheres, talc or tribasic calcium phosphate.

7. The compressed tablet, as recited in any one of Claims 1 to 6, wherein the lubricant comprises: calcium stearate, glyceryl monostearate, glyceryl palmitostearate, hydrogenated castor oil, hydrogenated vegetable oil, light mineral oil, magnesium stearate, mineral oil, polyethylene glycol, sodium benzoate, sodium lauryl sulfate, sodium stearyl fumarate, stearic acid, talc or zinc stearate.

8. The compressed tablet, as recited in any one of Claims 1 to 7, wherein the solvent comprises: water, ethanol or mixtures thereof.

9. The compressed tablet, as recited in Claim 8, wherein the filler/disintegrant is microcrystalline cellulose.

10. The compressed tablet, as recited in Claim 9, wherein the superdisintegrant is croscarmellose sodium.

11. The compressed tablet, as recited in Claim 10, wherein the binder is hydroxypropyl cellulose.

12. The compressed tablet, as recited in Claim 11, wherein the surfactant is sodium lauryl sulfate.

13. The compressed tablet, as recited in Claim 12, wherein the diluent/compression aid is lactose hydrous spray dried.

14. The compressed tablet, as recited in Claim 13, wherein the lubricant is magnesium stearate.

15. The compressed tablet, as recited in Claim 14, comprising efavirenz, microcrystalline cellulose NF, hydroxypropyl cellulose LF NF, croscarmellose sodium, sodium lauryl sulfate, lactose hydrous spray dried (EG), and magnesium stearate (EG).

16. The compressed tablet, as recited in Claim 15, containing about 300 mg of efavirenz, about 120 mg microcrystalline cellulose NF, about 19.2 mg hydroxypropyl cellulose LF NF, about 30 mg croscarmellose sodium, about 6 mg sodium lauryl sulfate, about 118.8 mg lactose hydrous spray dried (EG), and about 6 mg magnesium stearate (EG).

17. The compressed tablet as recited in any one of Claims 1 to 15, wherein efavirenz is present in an amount of 300 mg.

18. The compressed tablet as recited in any one of Claims 1 to 15, wherein efavirenz is present in an amount of 600 mg.

19. The compressed tablet as recited in any one of Claims 1 to 18, wherein efavirenz is about 50% by weight of the total composition of the compressed tablet.

20. The compressed tablet as recited in any one of Claims 1 to 19, wherein the compressed tablet is prepared via wet granulation in which efavirenz, filler/disintegrant, superdisintegrant, binder, and surfactant are blended intragranularly, and diluent/compression aid and lubricant are added extragranularly.

21. A process for the preparation of a 50% drug loaded compressed tablet comprising the following steps:
(a) blending efavirenz with a filler/disintegrant, super-disintegrant, binder and surfactant;
(b) adding at least 1.1% by weight of water per weight of efavirenz to wet granulate the blended mixture to agglomerate the mixture;
(c) drying the granulated mixture to a moisture content of up to about 10%;
(d) milling the dried mixture to granulate to a uniform size;
(e) blending the milled mixture with a diluent/compression aid;
(f) lubricating the blended mixture with a lubricant; and
(g) compressing the lubricated mixture to a compressed tablet of the desired shape.

22. The process as recited in Claim 21 which comprises the additional step of film coating the compressed tablet with a film coating suspension to produce the desired film coated compressed tablet.

23. The process as recited in Claim 21 or 22 wherein the granulated mixture is dried to a moisture content of from 2% to 5%.

24. A process for the preparation of a 50% drug loaded film coated compressed tablet comprising the following steps:
(a) blending efavirenz with microcrystalline cellulose, sodium lauryl sulfate, hydroxypropyl cellulose and croscarmellose sodium;
(b) adding at least 1.1 weight % water per weight of efavirenz to wet granulate the blended mixture for from 3 minutes to 8 minutes to agglomerate the mixture;
(c) drying the granulated mixture to a moisture content of from 2% to 5%.
(d) milling the dried mixture to a granulate of from 250µ to 75µ;
(e) blending the milled mixture with lactose;
(f) lubricating the blended mixture with magnesium stearate;
(g) compressing the lubricated mixture to a compressed tablet of the desired shape; and
(h) film coating the compressed tablet with a film coating suspension from 1% to 10% by weight of the weight of compressed tablet.

25. The process as recited Claim 24, wherein the blended mixture is wet granulated for about 6 minutes.

26. The process as recited Claim 24 or 25, wherein the film coating suspension comprises hydroxypropylcellulose, hydroxypropyl methylcellulose, and titanium dioxide.

27. The process as recited Claim 24, 25 or 26, wherein the compressed tablet is film coated with the film coating suspension to from 3.1% to 3.3% by weight of the weight of compressed tablet.

28. A compressed tablet comprising efavirenz, filler/disintegrant, superdisintegrant, binder, surfactant, diluent/compression aid, lubricant and solvent, wherein efavirenz is about 50% by weight of the total composition of the compressed tablet, obtainable by wet granulation in which the filler/disintegrant and superdisintegrant are added intragranularly and the diluent/compression aid is added extragranularly.

## Patentansprüche

1. Eine Preßtablette, die enthält: Efavirenz, Füllstoff/Sprengmittel, Supersprengmittel, Bindemittel, Surfactant, Verdünnungsmittel/Preßhilfsmittel, Gleitmittel und Lösungsmittel, wobei das Efavirenz kristallin ist und 1 bis 75 Gew.-% der Gesamtzusammensetzung der Preßtablette ausmacht.

2. Die wie in Anspruch 1 aufgeführte Preßtablette, wobei der Füllstoff umfaßt: Lactose, Calciumcarbonat, Calciumsulfat, verpreßbare Zucker, Dextrate, Dextrin, Dextrose, Calciumphosphat, Kaolin, Magnesiumcarbonat, Magnesiumoxid, Maltodextrinmannit, pulverförmige Cellulose, vorgelierte Stärke oder Saccharose.

3. Die wie in Anspruch 1 oder 2 aufgeführte Preßtablette, wobei das Sprengmittel und das Supersprengmittel umfassen: Alginsäure, Carboxymethylcellulose-Calcium, Carboxymethylcellulose-Natrium, kolloidales Siliciumdioxid, Croscarmellose-Natrium, Crospovidon, Guargummi, Magnesiumaluminiumsilicat, Methylcellulose, mikrokristalline Cellulose, Polacrilin-Kalium, pulverförmige Cellulose, vorgelierte Stärke, Natriumalginat oder Stärke.

4. Die wie in Anspruch 1, 2 oder 3 aufgeführte Preßtablette, wobei das Bindemittel umfaßt: Akaziengummi, Alginsäure, Carbomer, Dextrin, Ethylcellulose, Gelatine, Guargummi, Hydroxyethylcellulose, Hydroxypropylcellulose, Hydroxypropylmethylcellulose, flüssige Glucose, Magnesiumaluminiumsilicat, Maltodextrin, Methylcellulose, Polymethacrylate, Povidon, vorgelierte Stärke, Natriumalginat, Stärke oder Zein.

5. Die wie in Anspruch 1, 2, 3 oder 4 aufgeführte Preßtablette, wobei der Surfactant umfaßt: Natriumlaurylsulfat, Docusat-Natrium, Benzalkoniumchlorid, Benzethoniumchlorid oder Cetrimid.

6. Die wie in irgendeinem der Ansprüche 1 bis 5 aufgeführte Preßtablette, wobei das Verdünnungsmittel/Preßhilfsmittel umfaßt: Natriumcarbonat, Calciumsulfat, verpreßbare Zucker, Puderzucker, Dextrate, Dextrin, Dextrose, zweibasisches Calciumphosphat-Dihydrat, Glycerylpalmitostearat, hydriertes Pflanzenöl (Typ I), Kaolin, Lactose, wie z.B. wäßrige sprühgetrocknete Lactose, Magnesiumcarbonat, Magnesiumoxid, Maltodextrin, Mannit, Polymethacrylate, Kaliumchlorid, pulverförmige Cellulose, vorgelierte Stärke, Natriumchlorid, Sorbit, Stärke, Saccharose, Zuckerkügelchen, Talk oder dreibasisches Calciumphosphat.

7. Die wie in irgendeinem der Ansprüche 1 bis 6 aufgeführte Preßtablette, wobei das Gleitmittel umfaßt: Calciumstearat, Glycerylmonostearat, Glycerylpalmitostearat, hydriertes Rizinusöl, hydriertes Pflanzenöl, leichtes Mineralöl, Magnesiumstearat, Mineralöl, Polyethylenglycol, Natriumbenzoat, Natriumlaurylsulfat, Natriumstearylfumarat, Stearinsäure, Talk oder Zinkstearat.

8. Die wie in irgendeinem der Ansprüche 1 bis 7 aufgeführte Preßtablette, wobei das Lösungsmittel umfaßt: Wasser, Ethanol oder Mischungen davon.

9. Die wie in Anspruch 8 aufgeführte Preßtablette, wobei der Füllstoff/das Sprengmittel mikrokristalline Cellulose ist.

10. Die wie in Anspruch 9 aufgeführte Preßtablette, wobei das Supersprengmittel Croscarmellose-Natrium ist.

11. Die wie in Anspruch 10 aufgeführte Preßtablette, wobei das Bindemittel Hydroxypropylcellulose ist.

12. Die wie in Anspruch 11 aufgeführte Preßtablette, wobei der Surfactant Natriumlaurylsulfat ist.

13. Die wie in Anspruch 12 aufgeführte Preßtablette, wobei das Verdünnungsmittel/Preßhilfsmittel wäßrige sprühgetrocknete Lactose ist.

14. Die wie in Anspruch 13 aufgeführte Preßtablette, wobei das Gleitmittel Magnesiumstearat ist.

15. Die wie in Anspruch 14 aufgeführte Preßtablette, die Efavirenz, mikrokristalline Cellulose NF, Hydroxypropylcellulose LF NF, Croscarmellose-Natrium, Natriumlaurylsulfat, wäßrige sprühgetrocknete Lactose (EG) und Magnesiumstearat (EG) enthält.

16. Die wie in Anspruch 15 aufgeführte Preßtablette, die etwa 300 mg Efavirenz, etwa 120 mg mikrokristalline Cellulose NF, etwa 19,2 mg Hydroxypropylcellulose LF NF, etwa 30 mg Croscarmellose-Natrium, etwa 6 mg Natriumlaurylsulfat, etwa 118,8 mg wäßrige sprühgetrocknete Lactose (EG) und etwa 6 mg Magnesiumstearat (EG) enthält.

17. Die wie in irgendeinem der Ansprüche 1 bis 15 aufgeführte Preßtablette, wobei Efavirenz in einer Menge von 300 mg vorliegt.

18. Die wie in irgendeinem der Ansprüche 1 bis 15 aufgeführte Preßtablette, wobei Efavirenz in einer Menge von 600 mg vorliegt.

19. Die wie in irgendeinem der Ansprüche 1 bis 18 aufgeführte Preßtablette, wobei Efavirenz etwa 50 Gew.-% der Gesamtzusammensetzung der Preßtablette ausmacht.

20. Die wie in irgendeinem der Ansprüche 1 bis 19 aufgeführte Preßtablette, wobei die Preßtablette durch Naßgranulation hergestellt wird, bei der Efavirenz, Füllstoff/Sprengmittel, Supersprengmittel, Bindemittel und Surfactant intragranular vermischt werden und das Verdünnungsmittel/Preßhilfsmittel und das Gleitmittel extragranular zugegeben werden.

21. Ein Verfahren zur Herstellung einer mit 50% Arzneistoff angereicherten Preßtablette, umfassend die folgenden Schritte:
(a) Vermischen von Efavirenz mit einem Füllstoff/Sprengmittel, Supersprengmittel, Bindemittel und Surfactant,
(b) Zugabe von wenigstens 1,1 Gew.-% Wasser pro Gewicht Efavirenz, um die vermischte Mischung naß zu granulieren, um die Mischung zu agglomerieren,
(c) Trocknen der granulierten Mischung bis zu einem Feuchtigkeitsgehalt von bis zu etwa 10%,
(d) Mahlen der getrockneten Mischung, um sie zu einer einheitlichen Größe zu granulieren,
(e) Vermischen der gemahlenen Mischung mit einem Verdünnungsmittel/Preßhilfsmittel,
(f) Gleitfähigmachen der vermischten Mischung mit einem Gleitmittel und
(g) Verpressen der gleitfähig gemachten Mischung zu einer Preßtablette mit der erwünschten Form.

22. Das wie in Anspruch 21 aufgeführte Verfahren, das den zusätzlichen Schritt der Filmbeschichtung der Preßtablette mit einer Filmbeschichtungssuspension umfaßt, um die erwünschte filmbeschichtete Preßtablette zu ergeben.

23. Das wie in Anspruch 21 oder 22 aufgeführte Verfahren, wobei die granulierte Mischung bis zu einem Feuchtigkeitshalt von 2% bis 5% getrocknet wird.

24. Ein Verfahren zur Herstellung einer mit 50% Arzneistoff angereicherten filmbeschichteten Preßtablette, umfassend die folgenden Schritte:
(a) Vermischen von Efavirenz mit mikrokristalliner Cellulose, Natriumlaurylsulfat, Hydroxypropylcellulose und Croscarmellose-Natrium,
(b) Zugabe von wenigstens 1,1 Gew.-% Wasser pro Gewicht Efavirenz, um die vermischte Mischung 3 Minuten bis 8 Minuten lang naß zu granulieren, um die Mischung zu agglomerieren,
(c) Trocknen der granulierten Mischung bis zu einem Feuchtigkeitsgehalt von 2% bis 5%,
(d) Mahlen der getrockneten Mischung bis zu einem Granulat mit 250 µ bis 75 µ,
(e) Vermischen der gemahlenen Mischung mit Lactose,
(f) Gleitfähigmachen der vermischten Mischung mit Magnesiumstearat,
(g) Verpressen der gleitfähig gemachten Mischung zu einer Preßtablette mit der erwünschten Form und
(h) Filmbeschichten der Preßtablette mit einer Filmbeschichtungssuspension mit 1 Gew.-% bis 10 Gew.-% des Gewichts der Preßtablette.

25. Das wie in Anspruch 24 aufgeführte Verfahren, wobei die vermischte Mischung etwa 6 Minuten lang naßgranuliert wird.

26. Das wie in Anspruch 24 oder 25 aufgeführte Verfahren, wobei die Filmbeschichtungssuspension Hydroxypropylcellulose, Hydroxypropylmethylcellulose und Titandioxid umfaßt.

27. Das wie in Anspruch 24, 25 oder 26 aufgeführte Verfahren, wobei die Preßtablette mit der Filmbeschichtungssuspension filmbeschichtet wird, um 3,1 Gew.-% bis 3,3 Gew.-% des Gewichts der Preßtablette zu ergeben.

28. Eine Preßtablette, die Efavirenz, Füllstoff/Sprengmittel, Supersprengmittel, Bindemittel, Surfactant, Verdünnungsmittel/Preßhilfsmittel, Gleitmittel und Lösungsmittel enthält, wobei das Efavirenz etwa 50 Gew.-% der Gesamtzusammensetzung der Preßtablette ausmacht, erhältlich durch Naßgranulation, wobei der Füllstoff/das Sprengmittel und das Supersprengmittel intragranular und das Verdünnungsmittel/Preßhilfsmittel extragranular zugegeben werden.

## Revendications

1. Comprimé comprenant : de l'éfavirenz, une charge/un délitant, un superdélitant, un liant, un tensioactif, un diluant/adjuvant de compression, un lubrifiant et un solvant, dans lequel l'éfavirenz est cristallin et représente de 1 à 75% en poids de la composition totale du comprimé.

2. Comprimé selon la reveadication 1, dans lequel la charge comprend : du lactose, du carbonate de calcium, du sulfate de calcium, des sucres compressibles, des dextrates, de la dextrine, du dextrose, du phosphate de calcium, du kaolin, du carbonate de magnésium, de l'oxyde de magnésium, de la maltodextrine, du mannitol, de la cellulose en poudre, de l'amidon prégélatinisé ou du saccharose.

3. Comprimé selon la revendication 1 ou 2, dans lequel le délitant et le superdélitant comprennent : de l'acide alginique, de la carboxymétbylcellulose calcique, de la carboxyméthylcellulose sodique, du dioxyde de silicium colloïdal, de la croscarmellose sodique, de la crospovidone, de la gomme de guar, de l'aluminosilicate de magnésium, de la méthylcellulose, de la cellulose microcristalline, de la polyacriline potassique, de la cellulose en poudre, de l'amidon prégélatinisé, de l'alginate de sodium ou de l'amidon.

4. Comprimé selon la revendication 1, 2 ou 3, dans lequel le liant comprend : de la gomme arabique, de l'acide alginique, du carbomère, de la dextrine, de l'éthylcellulose, de la gélatine, de la gomme de guar, de l'hydroxyéthylcellulose, de l'hydroxypropylcellulose, de l'hydroxypropylméthylcellulose, du glucose liquide, de l'aluminosilicate de magnésium, de la maltodextrine, de la méthylcellulose, des polyméthacrylates, de la povidone, de l'amidon prégélatinisé, de l'alginate de sodium, de l'amidon ou de la zéine.

5. Comprimé selon la revendication 1, 2, 3 ou 4, dans lequel le tensioactif comprend : du laurylsulfate de sodium, du docusate sodique, du chlorure de benzalkonium, du chlorure de benzéthonium ou du cétrimide.

6. Comprimé selon l'une quelconque des revendications 1 à 5, dans lequel le diluant/adjuvant de compression comprend : du carbonate de calcium, du sulfate de calcium, des sucres compressibles, du sucre glace, des dextrates, de la dextrine, du dextrose, du phosphate de dicalcium dihydraté, du palmitostéarate de glycéryle, de l'huile végétale hydrogénée (type I), du kaolin, du lactose, tel que du lactose hydraté séché par atomisation, du carbonate de magnésium, de l'oxyde de magnésium, de la maltodextrine, du mannitol, des polyméthacrylates, du chlorure de potassium, de la cellulose en poudre, de l'amidon prégélatinisé, du chlorure de sodium, du sorbitol, de l'amidon, du saccharose, des sphères de sucre, du talc ou du phosphate de tricalcium.

7. Comprimé selon l'une quelconque des revendications 1 à 6, dans lequel le lubrifiant comprend : du stéarate de calcium, du monostéarate de glycéryle, du palmitostéarate de glycéryle, de l'huile de ricin hydrogénée, de l'huile végétale hydrogénée, de l'huile minérale légère, du stéarate de magnésium, de l'huile minérale, du polyéthylèneglycol, du benzoate de sodium, du laurylsulfate de sodium, du stéarylfumarate de sodium, de l'acide stéarique, du talc ou du stéarate de zinc.

8. Comprimé selon l'une quelconque des revendications 1 à 7, dans lequel le solvant comprend : de l'eau, de l'éthanol ou leurs mélanges.

9. Comprimé selon la revendication 8, dans lequel la charge/le délitant est de la cellulose microcristalline.

10. Comprimé selon la revendication 9, dans lequel le superdélitant est de la croscarmellose sodique.

11. Comprimé selon la revendication 10, dans lequel le liant est de l'hydroxypropylcellulose.

12. Comprimé selon la revendication 11, dans lequel le tensioactif est du laurylsulfate de sodium.

13. Comprimé selon la revendication 12, dans lequel le diluant/adjuvant de compression est du lactose hydraté séché par atomisation.

14. Comprimé selon la revendication 13, dans lequel le lubrifiant est du stéarate de magnésium.

15. Comprimé selon la revendication 14, comprenant de l'éfavirenz, de la cellulose microcristalline NF, de l'hydroxypropylcellulose LF NF, de la croscannellose sodique, du laurylsulfate de sodium, du lactose hydraté séché par atomisation (EG) et du stéarate de magnésium (EG).

16. Comprimé selon la revendication 15, contenant environ 300 mg d'éfavirenz, environ 120 mg de cellulose microcristalline NF, environ 19,2 mg d'hydroxypropylcellulose LF NF, environ 30 mg de croscarmellose sodique, environ 6 mg de laurylsulfate de sodium, environ 118,8 mg de lactose hydraté séché par atomisation (EG) et environ 6 mg de stéarate de magnésium (EG).

17. Comprimé selon l'une quelconque des revendications 1 à 15, dans lequel l'éfavirenz est présent à raison de 300 mg.

18. Comprimé selon l'une quelconque des revendications 1 à 15, dans lequel l'éfavirenz est présent à raison de 600 mg.

19. Comprimé selon l'une quelconque des revendications 1 à 18, dans lequel l'éfavirenz représente environ 50% du poids de la composition totale du comprimé.

20. Comprimé selon l'une quelconque des revendications 1 à 19, dans lequel le comprimé est préparé par une granulation par voie humide dans laquelle on incorpore dans les granulés l'éfavirenz, la charge/le délitant, le superdélitant, le liant et le tensioactif et on ajoute hors des granulés le diluant/adjuvant de compression et le lubrifiant.

21. Procédé de préparation d'un comprimé chargé à 50% de médicament, comprenant les étapes suivantes consistant à :
(a) mélanger l'éfavirenz avec une charge/un délitant, un superdélitant, un liant et un tensioactif ;
(b) ajouter au moins 1,1% en poids d'eau par rapport au poids d'éfarirenz, pour granuler par voie humide le mélange homogénéisé afin d'agglomérer le mélange ;
(c) sécher le mélange granulé jusqu'à une teneur en humidité allant jusqu'à environ 10% ;
(d) broyer le mélange séché pour effectuer la granulation jusqu'à une taille uniforme ;
(e) mélanger le mélange broyé avec un diluant/adjuvant de compression ;
(f) lubrifier le mélange homogénéisé avec un lubrifiant ; et
(g) compresser le mélange lubrifié pour obtenir un comprimé de forme voulue.

22. Procédé selon la revendication 21 qui comprend l'étape supplémentaire consistant à pelliculer le comprimé avec une suspension de pelliculage afin de produire le comprimé pelliculé recherché.

23. Procédé selon la revendication 21 ou 22 dans lequel le mélange granulé est séché jusqu'à une teneur en humidité de 2% à 5%.

24. Procédé de préparation d'un comprimé pelliculé chargé à 50% de médicament comprenant les étapes suivantes consistant à :
(a) mélanger l'éfavirenz avec de la cellulose microcristalline, du laurylsulfate de sodium, de l'hydroxypropylcellulose et de la croscarmellose sodique ;
(b) ajouter au moins 1,1% en poids d'eau par rapport au poids d'éfarirenz, pour granuler par voie humide le mélange homogénéisé pendant 3 minutes à 8 minutes afin d'agglomérer le mélange ;
(c) sécher le mélange granulé jusqu'à une teneur en humidité de 2% à 5% ;
(d) broyer le mélange séché pour obtenir un granulat de 250 µ à 75 µ ;
(e) mélanger le mélange broyé avec du lactose ;
(f) lubrifier le mélange homogénéisé avec du stéarate de magnésium ;
(g) compresser le mélange lubrifié pour obtenir un comprimé de forme voulue ; et
(h) pelliculer le comprimé d'une suspension de pelliculage à raison de 1% à 10% en poids, par rapport au poids du comprimé.

25. Procédé selon la revendication 24, dans lequel le mélange homogénéisé est granulé par voie humide pendant environ 6 minutes.

26. Procédé selon la revendication 24 ou 25, dans lequel la suspension de pelliculage comprend de l'hydroxypropylcellulose, de l'hydroxypropylméthylcellulose et du dioxyde de titane.

27. Procédé selon la revendication 24, 25 ou 26, dans lequel le comprimé est enrobé de la suspension de pelliculage à raison de 3,1% à 3,3% en poids, par rapport au poids du comprimé.

28. Comprimé comprenant de l'éfavirenz, une charge/un délitant, un superdélitant, un liant, un tensioactif, un diluant/adjuvant de compression, un lubrifiant et un solvant, dans lequel l'éfavirenz représente environ 50% du poids de la composition totale du comprimé, qui peut être obtenu par une granulation par voie humide dans laquelle la charge/le délitant et le superdélitant sont ajoutés dans les granulés, et le diluant/adjuvant de compression est ajouté hors des granulés.
